# EUROPÄISCHE PATENTSCHRIFT

(11) **EP 1 325 063 B1**
(45) Veröffentlichungstag und Bekanntmachung des Hinweises auf die Patenterteilung: **08.03.2006**
(21) Anmeldenummer: 01980412.9
(22) Anmeldetag: 19.09.2001
(51) Int. Cl.: C08J 9/28, C08G 12/26, C08L 61/32, A61L 15/26, C08J 9/36

(54) **HYDROPHILE, OFFENZELLIGE, ELASTISCHE SCHAUMSTOFFE AUF BASIS VON MELAMIN/FORMALDEHYD-HARZEN, IHRE HERSTELLUNG UND IHRE VERWENDUNG IN HYGIENEARTIKELN**
HYDROPHILIC, OPEN-CELL, ELASTIC FOAMS WITH A MELAMINE/FORMALDEHYDE RESIN BASE, PRODUCTION THEREOF AND USE THEREOF IN HYGIENE PRODUCTS
PRODUITS ALVEOLAIRES, HYDROPHILES, A ALVEOLES OUVERTES ET ELASTIQUES A BASE DE RESINES MELAMINE/FORMALDEHYDE, LEUR PRODUCTION ET LEUR UTILISATION DANS DES ARTICLES D'HYGIENE

(30) Priorität: 27.09.2000 DE 10047719
(43) Veröffentlichungstag der Anmeldung: 09.07.2003
(73) Patentinhaber: BASF Aktiengesellschaft, 67056 Ludwigshafen (DE)
(72) Erfinder: BAUMGARTL, Horst, 67063 Ludwigshafen (DE); HERFERT, Norbert, 63674 Altenstadt (DE); HÄHNLE, Hans-Joachim, 67435 Neustadt (DE)
(86) Internationale Anmeldenummer: PCT/EP2001/010846
(87) Internationale Veröffentlichungsnummer: WO 2002/026872

(56) Entgegenhaltungen:
- GB-A- 1 570 485

## Beschreibung

Die Erfindung betrifft hydrophile, offenzellige, elastische Schaumstoffe auf Basis von Melamin/Formaldehyd-Harzen, ihre Herstellung und ihre Verwendung in Hygieneartikeln.

Aus der EP-A-0 017 621 und EP-A-0 017 672 sind offenzellige, elastische Schaumstoffe auf Basis von Melamin/Formaldehyd-Kondensationsprodukten'sowie Verfahren zu ihrer Herstellung bekannt. Nach dem aus der EP-A-0 037 470 bekannten Verfahren erfolgt die Herstellung von offenzelligen, elastischen Schaumstoffen aus Melamin/Formaldehyd-Kondensationsprodukten besonders vorteilhaft durch Einwirkung von Mikrowellenenergie (Ultrahochfrequenzbestrahlung) auf eine wäßrige Lösung oder Dispersion, die jeweils ein Melamin/Formaldehyd-Vorkondensat, einen Emulgator, ein Treibmittel und einen Härter enthalten. Die Lösung bzw. Dispersion wird dabei derart erhitzt, daß sie aufschäumt und das Vorkondensat aushärtet. Die so erhältlichen Schaumstoffe emittieren geringe Mengen an Formaldehyd, wobei die Formaldehyd-Emission mit zunehmender Temperatur und Feuchte des Schaumstoffs ansteigt.

Der Aufbau von Hygieneartikeln und die Verwendung von offenzelligen Schaumstoffen aus Melamin/Formaldehyd-Harzen als absorbierende Zwischenschicht werden in der älteren, nicht vorveröffentlichten DE-Anmeldung Nr. 100 34 505.0 ausführlich beschrieben. Die dort angeführten Schaumstoffe aus Melamin/Formaldehyd-Harzen sind zwar hochgradig hydrophil, weisen aber eine verhältnismäßig hohe Formaldehydabgabe bei Kontakt mit Körperflüssigkeiten auf. Dadurch werden die Einsatzmöglichkeiten derartiger Schaumstoffe in Hygieneartikeln erheblich eingeschränkt.

Aus der älteren, nicht vorveröffentlichten DE-Anmeldung Nr. 100 27 770.5 ist die Herstellung von Schaumstoffen aus formaldehydarmen, offenzelligen Melamin/Formaldehyd-Harzen mit einem Molverhältnis von Melamin/Formaldehyd von 1 : 1,0 bis 1 : 1,9 beschrieben. Diese Schaumstoffe emittieren selbst unter den im Hygienebereich üblichen warm / feucht Bedingungen weniger als 30 mg Formaldehyd/kg Schaumstoff (EU-Norm EN ISO 14 184-1, Wasserlagerung 1 h bei 40 Grad C). Sie erfüllen damit die an Babybekleidung gestellten Forderungen des ÖKOTEX-Standard 100 (Qualitätssiegel der Textilindustrie für besonders schadstoffarme Textilien). Die erhebliche Reduktion der Formaldehyd-Emission wird jedoch mit einem partiellen Verlust der hydrophilen Eigenschaften des Schaumstoffes erkauft, wodurch die Flüssigkeitsaufnahmegeschwindigkeit solcher Schaumstoffschichten absinkt.

Aus der WO-A-96/21682 sind Schaumstoffe bekannt, die aufgrund ihrer offenzelligen Struktur mit relativ großen Öffnungen und Kanälen hervorragend zur Absorption wäßriger Körperflüssigkeiten, insbesondere zur Blutabsorption geeignet sind. Die Schaumstoffe werden erhalten durch Polymerisation von (C₄-C₁₄)-Alkylacrylaten, (C₆-C₁₆)-Alkylmethacrylaten, (C₄-C₁₂)-Alkylstyrolen als Monomere, bevorzugt Styrol und Ethylstyrol als Comonomere, des weiteren aromatische Polyvinylverbindungen als Vernetzer; optional polyfunktionelle Acrylate, Methacrylate, Acrylamide und Methacrylamide und Mischungen davon als zusätzliche Vernetzersubstanzen. Die Polymerisation findet innerhalb einer High-Internal-Phase-Emulsion (HIPE) vom Typ W/O statt, wobei das Gewichtsverhältnis von Wasserphase zu Ölphase 20 :1 bis 125 : 1 beträgt. Nach beendeter Polymerisation erfolgt das Waschen und Trocknen der Polymerschäume.

WO-A-97/07832, US-A-5,318,554 und US-A-5,550,167 betreffen die Herstellung offenzelliger Schaumstoffe auf Basis von HIPE-Emulsionen und deren Verwendung zur Absorption wäßriger Körperflüssigkeiten. Die offenzelligen Schäume werden allerdings immer zusammen mit anderen Komponenten eingesetzt, die im Hygieneartikel die endgültige Absorption (Immobilisierung) der Körperflüssigkeiten übernehmen. Die Materialien haben zwar gute anwendungstechnische Eigenschaften, aber weisen dennoch klare Nachteile auf.

So ist ihre Herstellung extrem aufwendig, weil der Prozess verfahrenstechnisch nur schwer zu kontrollieren ist. Der enorme Aufwand an wässriger Phase (wässrige Salzlösung) ist weder ökonomisch noch ökologisch sinnvoll. Die Materialien tragen außerdem zur Hydrophilierung an der Oberfläche eine Salzschicht. Diese Schicht kann während des Gebrauchs abgelöst und in das Speichermedium des absorbent core ausgewaschen werden. Als Speichermedium werden in der Regel Superabsorbern eingesetzt. Es ist bekannt, daß Superabsorber das Phänomen der "Salzvergiftung" zeigen, d.h. ihre Aufnahmekapazität geht mit steigendem Salzgehalt der zu absorbierenden Lösung dramatisch zurück. Daher ist es nachteilig, die Salzfracht in den zu absorbierenden Körperflüssigkeiten zusätzlich zu erhöhen.

Der vorliegenden Erfindung liegt daher die Aufgabe zugrunde, offenzellige, elastische Schaumstoffe auf Basis von Melamin/Formaldehydharzen zur Verfügung zu stellen, die hydrophil sind und deren Formaldehyd-Emission gegenüber bekannten Schaumstoffen aus Melamin/Formaldehydharzen deutlich erniedrigt ist.

Die Aufgabe wird erfindungsgemäß gelöst mit hydrophilen, offenzelligen, elastischen Schaumstoffen aus Melamin/Formaldehyd-Harzen, die erhältlich sind durch
(a) Erhitzen und Vernetzen einer wäßrigen Lösung oder Dispersion, die jeweils mindestens ein Melamin/Formaldehyd-Vorkondensat, einen Emulgator, ein Treibmittel und einen Härter enthalten, unter Ausbildung eines Schaumstoffs,
(b) anschließendes Tempern des Schaumstoffs während einer Zeit von 1 bis 180 Minuten bei 120 bis 300°C, wobei flüchtige Anteile entfernt werden, und
(c) Behandeln des Schaumstoffs während des Temperns oder danach mit mindestens einem Polymeren, das primäre und/oder sekundäre Aminogruppen enthält und eine Molmasse von mindestens 300 hat.

Für die Herstellung der Schaumstoffe setzt man beispielsweise Melamin/Formaldehyd-Vorkondensate ein, bei denen das Molverhältnis Melamin zu Formaldehyd 1 : 1,0 bis 1 : 5,0 beträgt. Bevorzugt werden solche Melamin/Formaldehyd-Vorkondensate, bei denen das Molverhältnis Melamin zu Formaldehyd 1 : 2,0 bis 1 : 5,0 und insbesondere 1 : 2,5 bis 1 : 3,5 beträgt. Die Molmasse der Polymeren, die primäre und/oder sekundäre Aminogruppen enthalten und die zur Modifizierung auf den hydrophilen, offenzelligen, elastischen Schaumstoffen vorhanden sind, beträgt beispielsweise 500 bis 5 Millionen, vorzugsweise 1000 bis 100 000. Diese Schaumstoffe enthalten vorzugsweise zur Verringerung der Formaldehyd-Emission und gegebenenfalls zur Hydrophilierung als Polymere Vinylamineinheiten enthaltende Polymerisate, Polyethylenimine, Polyallylamine, Lysinkondensate oder deren Mischungen.

Solche Schaumstoffe haben beispielsweise eine Dichte von 5 bis 200 g/l, eine spezifische Oberfläche (bestimmt nach BET) von mehr als 0,5 m²/g und eine Free Swell Capacity von mehr als 20 g/g. Sie weisen z.B. im nassen Zustand eine Zugfestigkeit von >60 J/m2 auf.

Gegenstand der Erfindung ist außerdem ein Verfahren zur Herstellung von hydrophilen, offenzelligen, elastischen Schaumstoffen, wobei man
(a) eine wäßrige Lösung oder Dispersion, die jeweils mindestens ein Melamin/Formaldehyd-Vorkondensat, einen Emulgator, ein Treibmittel und einen Härter enthalten, unter Ausbildung eines Schaumstoffs und Vernetzen des Vorkondensates erhitzt,
(b) den Schaumstoff dann während einer Zeit von 1 bis 180 Minuten bei 120 bis 300°C tempert, wobei flüchtige Anteile entfernt werden, und
(c) ihn während des Temperns oder danach mit mindestens einem Polymeren behandelt, das primäre und/oder sekundäre Aminogruppen enthält und eine Molmasse von mindestens 300 hat.

Die Verfahrensschritte (a) und (b) sind aus dem Stand der Technik bekannt, vgl. die oben bereits abgehandelten Literaturstellen EP-A-0 017 621, EP-A-0 017 672 und EP-A-0 037 470. Das Verschäumen gemäß dem Verfahrensschritt (a) geschieht durch Erhitzen der Mischung auf eine Temperatur oberhalb des Siedepunktes des Treibmittels und wird beispielsweise so durchgeführt wird, daß zunächst ein geringer Viskositätsanstieg erfolgt, und der Vernetzungsvorgang unter starker Viskositätserhöhung im wesentlichen erst dann einsetzt, wenn der Schäumvorgang beendet ist. Schäumen der Mischung und Vernetzen des Vorkondensats können jedoch auch gleichzeitig erfolgen. Das Erhitzen der Mischung wird beispielsweise durch Einwirkung von Heißluft, Wasserdampf und/oder Ausnutzen von Reaktionswärme vorgenommen. Das Aufschäumen der wäßrigen Mischung aus Melamin/Formaldehyd-Vorkondensat, Emulgator, Treibmittel und Härter geschieht vorzugsweise durch Einwirkung von Mikrowellenenergie nach dem aus der EP-A-0 0 37 470 bekannten Verfahren.

Struktur und mechanische Eigenschaften der Schaumstoffe sind aus der EP-A- 0 017 672 bekannt:
- Rohdichte nach DIN 53 420 liegt zwischen 1,6 und 30, vorzugsweise zwischen 2 und 20 [g/l]
- Wärmeleitzahl nach DIN 52 612 ist kleiner als 0,06, vorzugsweise kleiner als 0,04 [W.m⁻¹K⁻¹];
- Stauchhärte nach DIN 53 577 bei 60% Stauchung, dividiert durch die Rohdichte liegt unter 0,3, vorzugsweise unter 0,2 [N.cm⁻²/g.l⁻¹], wobei bei der Bestimmung der Stauchhärte bei 60% Stauchung eine Wiedererholung des Schaumstoffs auf 70%, vorzugsweise mindestens 90% und insbesondere 95% seiner ursprünglichen Abmessungen erfolgen muß.
- Elastizitätsmodul in Anlehnung an DIN 53 423, dividiert durch die Rohdichte liegt unter 0,25, vorzugsweise unter 0,15 [N.mm⁻²/g.l⁻¹].
- Biegeweg beim Bruch nach DIN 53 423 ist größer als 10, vorzugsweise größer als 15 [mm];
- Druckverformungsrest nach DIN 53 527 bei 50%iger Stauchung ist kleiner als 45%, vorzugsweise kleiner als 30% und insbesondere kleiner als 10%;
- Dynamische Steifigkeit nach DIN 18 165 bei einer Plattendicke von 50 mm ist kleiner als 20, vorzugsweise kleiner als 10 und insbesondere kleiner als 5 [N.cm⁻³];
- Entflammbarkeit nach DIN 4102: mindestens normalentflammbar, vorzugsweise schwerentflammbar
- Zugfestigkeit im nassen Zustand > 60 J/ m²
- Oberfläche des Schaums nach der BET-Methode > 0,5 m²/g.

Die Schaumstoffe auf Basis von Melamin/Formaldehyd-Kondensationsprodukten, die erfindungsgemäß eingesetzt werden, sind offenporig. Bei einer mikroskopischen Betrachtung der Schaumstoffe zeigt es sich, daß das Schaumgerüst eine Vielzahl miteinander verbundener, dreidimensional verzweigter Stege enthält. Melamin/Formaldehyd-Harzschäume sind beispielsweise dann ausreichend elastisch, wenn die Stege die in der EP-A- 0 017 672 beschriebenen Bedingungen erfüllen, d.h. das mittlere Verhältnis von Länge zu Dicke der Stege ist größer als 10 : 1, vorzugsweise größer als 12 : 1 und insbesondere größer als 15 : 1 und die Dichte der Stege mehr als 1,10, vorzugsweise mehr als 1,20 und insbesondere mehr als 1,30 g/cm³ beträgt. Länge und Dicke der Stege werden z.B. mikroskopisch bestimmt, die Dichte der Schaumstoffstege kann beispielsweise durch Tauchen der Schaumstoffe in eine geeignete Flüssigkeit wie Isopropanol nach dem archimedischen Prinzip bestimmt werden, vgl. EP-A-0 017 672.

Gemäß dem Verfahrensschritt (b) wird der Schaumstoff während einer Zeit von 1 bis 180 Minuten bei 120 bis 300°C getempert. Er wird dabei vorzugsweise 3 bis 60 Minuten lang auf Temperaturen von 120 und 260 °C, besonders bevorzugt von 150 bis 250 °C erhitzt, wobei Wasser, Treibmittel und Formaldehyd weitgehend entfernt werden und eine Nachhärtung des Schaumharzes erfolgt. Diese Temperaturbehandlung kann unmittelbar anschließend an die Schaumherstellung in derselben Apparatur oder in einer nachgeschalteten Apparatur erfolgen; sie kann aber auch zu einem späteren Zeitpunkt unabhängig vom Schäumprozeß durchgeführt werden. Getemperte Schaumstoffe zeigen eine wesentlich geringere Neigung zum Schwinden und weisen eine geringere Gleichgewichtsfeuchte auf als ungetemperte Produkte. Auch die Formaldehyd-Emission der getemperten Schaumstoffe ist gegenüber der Formaldehydemission der ungetemperten Produkte stark verringert. Die Formaldehydabspaltung beträgt weniger als 100 mg Formaldehyd / kg Schaumstoff, bevorzugt weniger als 20 mg Formaldehyd / kg Schaumstoff (gemessen nach der EU Norm ISO 14184-1).

Die Schaumstoffe können als Platten, Blöcke oder Bahnen mit einer Höhe von bis zu 2 m hergestellt werden oder als Schaumfolien mit einer Dicke von wenigen mm, z.B. 0,5 bis 7mm. Die bevorzugte Schaumhöhe (in Schäumrichtung) liegt bei Verwendung von Mikrowellen der Frequenz 2,45 GHz zwischen 10 cm und 100 cm. Aus derartigen Schaumstoffblöcken können alle erwünschten Platten- bzw. Vliesstärken herausgeschnitten werden.

Um die Formaldehydemission der Schaumstoffe weiter zu senken und um gegebenenfalls die Aufnahmegeschwindigkeit der Schaumstoffe für Wasser und Körperflüssigkeiten zu erhöhen, werden sie im Verfahrensschritt (c) entweder während des Temperns oder danach mit mindestens einem Polymeren behandelt, das primäre und/oder sekundäre Aminogruppen enthält und eine Molmasse von mindestems 300 hat. Die Behandlung des Schaumstoffs kann bereits während des Temperns durchgeführt werden, indem man beispielsweise den Melamin/Formaldehydharzschaumstoff zur Entfernung aller flüchtigen Bestandteile mit heißer Luft durchströmt und dieser Temperluft primäre und/oder sekundäre Aminogruppen enthaltende Polymere beispielsweise in Form eines Aerosols hinzufügt. Auf diese Weise kann die Forrmaldehydemission der Schaumstoffe gesenkt und gegebenenfalls eine Hydrophilierung erzielt werden, ohne die Schaumstoffe nachträglich einer weiteren Behandlung unterwerfen zu müssen.

Im Verfahrensschritt (c) erfolgt die Behandlung der Schaumstoffe mit mindestens einem Polymer, das primäre und/oder sekundäre Aminogruppen aufweist und eine Molmasse von mindestens 300 hat. Eine Behandlung der Oberfläche des Schaumstoffs kann auch durch Aufbringen von vernetzten Polymerisaten oder einer vernetzten, hydrophilen Hülle erfolgen, indem man z.B. zunächst primäre und/oder sekundäre Aminogruppen enthaltende Polymere auf die Oberfläche der Schaumstoffe aufbringt, dann darauf einen Vernetzer dosiert und ihn mit den funktionellen Gruppen der Polymeren reagieren läßt.

Die polymeren Behandlungsmittel werden normalerweise in gelöster Form angewandt, indem sie in einem Lösemittel gelöst werden. Sie können auch in Form von wäßrigen Dispersionen oder Dispersionen in einem organischen Lösemittel auf die zu Melamin/Formaldehydharz- Schaumstoffe appliziert werden. Die Behandlung kann z.B. durch Eintauchen des Melamin/Formaldehyd-Schaumstoffkörpers in die Flüssigkeit erfolgen, die mindestens ein primäre und/oder sekundäre Aminogruppen enthaltendes Poylmer in gelöster oder in dispergierter Form enthält. Alternativ kann die Flüssigkeit mit dem gelösten oder dem dispergierten polymeren Behandlungsmittel auch durch Aufsprühen auf die Schaumstoffoberfläche appliziert werden. Danach wird das Lösemittel aus dem so behandelten Schaumstoffkörper entfernt, z.B. durch Trocknen des Schaumstoffs.

Das Aminogruppen enthaltende Polymer reagiert mit dem so hergestellten Melamin/Formaldehydharzschaum bzw. wird an den Polymeroberflächen adsorbiert. Die Menge an zugesetztem polymeren Behandlungsmittel wird so bemessen, daß die Formaldehyd-Emission des Schaums so weit erniedrigt wird, daß sie weniger als 100 mg Formaldehyd/kg Schaumstoff beträgt. Die mechanischen Eigenschaften des Schaumstoffes (Flexibilität) sollen durch die Modifizierung der Schaumstoffoberfläche mit den Polymeren nicht beeinträchtigt werden. Bevorzugt wird das polymere Behandlungsmittel in einer solchen Menge zugesetzt, daß die resultierende Menge an den Polymeren 0,1 bis 150 Gew.-%, vorzugsweise 0,5 bis 90 Gew.-% und insbesondere 1 bis 40 Gew.-%, bezogen auf den Schaumstoff, beträgt.

Als polymere Behandlungsmittel können sämtliche kationische synthetische Polymeren verwendet werden, die primäre und/oder sekundäre Amino- und/oder Ammoniumgruppen enthalten. Beispiele für solche kationische Polymere sind Vinylamineinheiten enthaltende Polymere, vernetzte Polyamidoamine, mit Ethylenimin gepfropfte vernetzte Polyamidoamine, Polyethylenimine, alkoxylierte Polyethylenimine, vernetzte Polyethylenimine, amidierte Polyethylenimine, alkylierte Polyethylenimine, Polyamine, Amin-Epichlorhydrin-Polykondensate, wasserlösliche Polyadditionsprodukte aus multifunktionellen Epoxiden und multifunktionellen Aminen, alkoxylierte Polyamine, Polyallylamine, und/oder Kondensate von Lysin, Ornithin oder Arginin.

Zur Herstellung von Vinylamineinheiten enthaltenden Polymerisaten geht man beispielsweise von offenkettigen N-Vinylcarbonsäureamiden der Formel 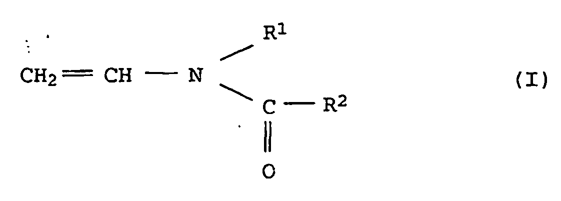
aus, in der R¹ und R² gleich oder verschieden sein können und für Wasserstoff und C₁- bis C₆-Alkyl stehen. Geeignete Monomere sind beispielsweise N-Vinylformamid (R¹=R²=H in Formel I) N-Vinyl-N-methylformamid, N-Vinylacetamid, N-Vinyl-N-methylacetamid, N-Vinyl-N-ethylacetamid, N-Vinyl-N-methylpropionamid und N-Vinylpropionamid. Zur Herstellung der Polymerisate können die genannten Monomeren entweder allein, in Mischung untereinander oder zusammen mit anderen monoethylenisch ungesättigten Monomeren polymerisiert werden. Vorzugsweise geht man von Homo- oder Copolymerisaten des N-Vinylformamids aus. Vinylamineinheiten enthaltende Polymerisate sind beispielsweise aus US-A-4 421 602, US-A-5 334 287, EP-A-0 216 387 und EP-A-0 251 182 bekannt. Sie werden durch Hydrolyse von Polymerisaten, die die Monomeren der Formel I einpolymerisiert enthalten, mit Säuren, Basen oder Enzymen erhalten.

Als monoethylenisch ungesättigte Monomere, die mit den N-Vinylcarbonsäureamiden copolymerisiert werden, kommen alle damit copolymerisierbaren Verbindungen in Betracht. Beispiele hierfür sind Vinylester von gesättigten Carbonsäuren von 1 bis 6 Kohlenstoffatomen wie Vinylformiat, Vinylacetat, Vinylpropionat und Vinylbutyrat und Vinylether wie C₁- bis C₆-Alkylvinylether, z.B. Methyl-oder Ethylvinylether. Weitere geeignete Comonomere sind ethylenisch ungesättigte C₃- bis C₆-Carbonsäuren, beispielsweise Acrylsäure, Methacrylsäure, Maleinsäure, Crotonsäure, Itaconsäure und Vinylessigsäure sowie deren Alkalimetall- und Erdalkalimetallsalze, Ester, Amide und Nitrile der genannten Carbonsäuren, beispielsweise Methylacrylat, Methylmethacrylat, Ethylacrylat und Ethylmethacrylat.

Weitere geeignete Carbonsäureester leiten sich von Glykolen oder bzw. Polyalkylenglykolen ab, wobei jeweils nur eine OH-Gruppe verestert ist, z.B. Hydroxyethylacrylat, Hydroxyethylmethacrylat, Hydroxypropylacrylat, Hydroxybutylacrylat, Hydroxypropylmethacrylat, Hydroxybutylmethacrylat sowie Acrylsäuremonoester von Polyalkylenglykolen einer Molmasse von 500 bis 10000. Weitere geeignete Comonomere sind Ester von ethylenisch ungesättigten Carbonsäuren mit Aminoalkoholen wie beispielsweise Dimethylaminoethylacrylat, Dimethylaminoethylmethacrylat, Diethylaminoethylacrylat, Diethylaminoethylmethacrylat, Dimethylaminopropylacrylat, Dimethylaminopropylmethacrylat, Diethylaminopropylacrylat, Dimethylaminobutylacrylat und Diethylaminobutylacrylat. Die basischen Acrylate können in Form der freien Basen, der Salze mit Mineralsäuren wie Salzsäure, Schwefelsäure oder Salpetersäure, der Salze mit organischen Säuren wie Ameisensäure, Essigsäure, Propionsäure oder der Sulfonsäuren oder in quaternierter Form eingesetzt werden. Geeignete Quaternierungsmittel sind beispielsweise Dimethylsulfat, Diethylsulfat, Methylchlorid, Ethylchlorid oder Benzylchlorid.

Weitere geeignete Comonomere sind Amide ethylenisch ungesättigter Carbonsäuren wie Acrylamid, Methacrylamid sowie N-Alkylmono- und Diamide von monoethylenisch ungesättigten Carbonsäuren mit Alkylresten von 1 bis 6 C-Atomen, z.B. N-Methylacrylamid, N,N-Dimethylacrylamid, N-Methylmethacrylamid, N-Ethylacrylamid, N-Propylacrylamid und tert. Butylacrylamid sowie basische (Meth)acrylamide, wie z.B. Dimethylaminoethylacrylamid, Dimethylaminoethylmethacrylamid, Diethylaminoethylacrylamid, Diethylaminoethylmethacrylamid, Dimethylaminopropylacrylamid, Diethylaminopropylacrylamid, Dimethylaminopropylmethacrylamid und Diethylaminopropylmethacrylamid.

Weiterhin sind als Comonomere geeignet N-Vinylpyrrolidon, N-Vinylcaprolactam, Acrylnitril, Methacrylnitril, N-Vinylimidazol sowie substituierte N-Vinylimidazole wie z.B. N-Vinyl-2-methylimidazol, N-Vinyl-4-methylimidazol, N-Vinyl-5-methylimidazol, N-Vinyl-2-ethylimidazol und N-Vinylimidazoline wie N-Vinylimidazolin, N-Vinyl-2-methylimidazolin und N-Vinyl-2-ethylimidazolin. N-Vinylimidazole und N-Vinylimidazoline werden außer in Form der freien Basen auch in mit Mineralsäuren oder organischen Säuren neutralisierter oder in quaternisierter Form eingesetzt, wobei die Quaternisierung vorzugsweise mit Dimethylsulfat, Diethylsulfat, Methylchlorid oder Benzylchlorid vorgenommen wird. In Frage kommen auch Diallyldialkylammoniumhalogenide wie z.B. Diallyldimethylammoniumchloride.

Außerdem kommen als Comonomere Sulfogruppen enthaltende Monomere wie beispielsweise Vinylsulfonsäure, Allylsulfonsäure, Methallylsulfonsäure, Styrolsulfonsäure, die Alkalimetall- oder Ammoniumsalze dieser Säuren oder Acrylsäure-3-sulfopropylester in Frage, wobei der Gehalt der amphoteren Copolymerisate an kationischen Einheiten den Gehalt an anionischen Einheiten übertrifft, so daß die Polymeren insgesamt eine kationische Ladung haben.

Die Copolymerisate enthalten beispielsweise
- 99,99 bis 1 mol-%, vorzugsweise 99,9 bis 5 mol-% N-Vinylcarbonsäureamide der Formel I und
- 0,01 bis 99 mol-%, vorzugsweise 0,1 bis 95 mol-% andere, damit copolymerisierbare monoethylenisch ungesättigte Monomere

in einpolymerisierter Form.

Um Vinylamineinheiten enthaltende Polymerisate herzustellen, geht man vorzugsweise von Homopolymerisaten des N-Vinylformamids oder von Copolymerisaten aus, die durch Copolymerisieren von
- N-Vinylformamid mit
- Vinylformiat, Vinylacetat, Vinylpropionat, Acrylnitril, N-Vinylcaprolactam, N-Vinylharnstoff, Acrylsäure, N-Vinylpyrrolidon oder C₁- bis C₆-Alkylvinylethern

und anschließende Hydrolyse der Homo- oder der Copolymerisate unter Bildung von Vinylamineinheiten aus den einpolymerisierten N-Vinylformamideinheiten erhältlich sind, wobei der Hydrolysegrad z. B. 0,1 bis 100 mol-% beträgt.

Die Hydrolyse der oben beschriebenen Polymerisate erfolgt nach bekannten Verfahren durch Einwirkung von Säuren, Basen oder Enzymen. Hierbei entstehen aus den einpolymerisierten Monomeren der oben angegebenen Formel I durch Abspaltung der Gruppierung 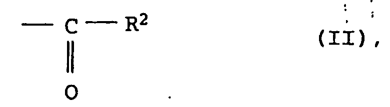
wobei R² die dafür in Formel I angegebene Bedeutung hat, Polymerisate, die Vinylamineinheiten der Formel 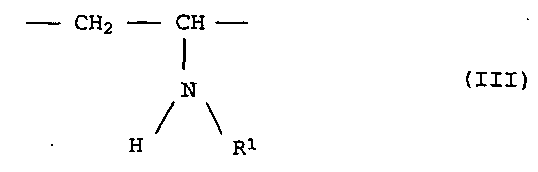
enthalten, in der R¹ die in Formel I angegebene Bedeutung hat. Bei Verwendung von Säuren als Hydrolysemittel liegen die Einheiten III als Ammoniumsalz vor.

Die Homopolymerisate der N-Vinylcarbonsäureamide der Formel I und ihre Copolymerisate können zu 0,1 bis 100, vorzugsweise 70 bis 100 mol-% hydrolysiert sein. In den meisten Fällen beträgt der Hydrolysegrad der Homo- und Copolymerisate 5 bis 95 mol-%. Der Hydrolysegrad der Homopolymerisate ist gleichbedeutend mit dem Gehalt der Polymerisate an Vinylamineinheiten. Bei Copolymerisaten, die Vinylester einpolymerisiert enthalten, kann neben der Hydrolyse der N-Vinylformamideinheiten eine Hydrolyse der Estergruppen unter Bildung von Vinylalkoholeinheiten eintreten. Dies ist insbesondere dann der Fall, wenn man die Hydrolyse der Copolymerisate in Gegenwart von Natronlauge durchführt. Einpolymerisiertes Acrylnitril wird ebenfalls bei der Hydrolyse chemisch verändert. Hierbei entstehen beispielsweise Amidgruppen oder Carboxylgruppen. Die Vinylamineinheiten enthaltenden Homo- und Copolymeren können gegebenenfalls bis zu 20 mol-% an Amidineinheiten enthalten, die z.B. durch Reaktion von Ameisensäure mit zwei benachbarten Aminogruppen oder durch intramolekulare Reaktion einer Aminogruppe mit einer benachbarten Amidgruppe z.B. von einpolymerisiertem N-Vinylformamid entsteht. Die Molmassen der Vinylamineinheiten enthaltenden Polymerisate betragen z.B. 500 bis 10 Millionen, vorzugsweise 1000 bis 5 Millionen (bestimmt durch Lichtstreuung). Dieser Molmassenbereich entspricht beispielsweise K-Werten von 5 bis 300, vorzugsweise 10 bis 250 (bestimmt nach H. Fikentscher in 5 %iger wäßriger Kochsalzlösung bei 25°C und einer Polymerkonzentration von 0,5 Gew.-%.

Die Vinylamineinheiten enthaltenden Polymeren werden vorzugsweise in salzfreier Form eingesetzt. Salzfreie wäßrige Lösungen von Vinylamineinheiten enthaltenden Polymerisaten können beispielsweise aus den oben beschriebenen salzhaltigen Polymerlösungen mit Hilfe einer Ultrafiltration an geeigneten Membranen bei Trenngrenzen von beispielsweise 1000 bis 500 000 Dalton, vorzugsweise 10 000 bis 300 000 Dalton hergestellt werden. Auch die unten beschriebenen wäßrigen Lösungen von Amino- und/oder Ammoniumgruppen enthaltenden anderen Polymeren können mit Hilfe einer Ultrafiltration in salzfreier Form gewonnen werden.

Polyethylenimine werden beispielsweise durch Polymerisation von Ethylenimin in wäßriger Lösung in Gegenwart von säureabspaltenden Verbindungen, Säuren oder Lewis-Säuren als Katalysator hergestellt. Polyethylenimine haben beispielsweise Molmassen bis zu 2 Millionen, vorzugsweise von 200 bis 1.000 000. Besonders bevorzugt werden Polyethylenimine mit Molmassen von 500 bis 750 000 eingesetzt. Außerdem eignen sich wasserlösliche vernetzte Polyethylenimine, die durch Reaktion von Polyethyleniminen mit Vernetzern wie Epichlorhydrin oder Bischlorhydrinethern von Polyalkylenglykolen mit 2 bis 100 Ethylenoxid- und/oder PropylenoxidEinheiten erhältlich sind und noch über freie primäre und/oder sekundäre Aminogruppen verfügen. Auch amidische Polyethylenimine sind geeignet, die beispielsweise durch Amidierung von Polyethyleniminen mit C₁- bis C₂₂-Monocarbonsäuren erhältlich sind. Weitere geeignete kationische Polymere sind alkylierte Polyethylenimine und alkoxylierte Polyethylenimine. Bei der Alkoxylierung verwendet man z.B. pro NH-Einheit in Polyethylenimin 1 bis 5 Ethylenoxid- bzw. Propylenoxideinheiten.

Geeignete primäre und/oder sekundäre Amino- und/oder Ammoniumgruppen enthaltende Polymere sind außerdem Polyamidoamine, die beispielsweise durch Kondensieren von Dicarbonsäuren mit Polyaminen erhältlich sind. Geeignete Polyamidoamine erhält man beispielsweise dadurch, daß man Dicarbonsäuren mit 4 bis 10 Kohlenstoffatomen mit Polyalkylenpolyaminen umsetzt, die 3 bis 10 basische Stickstoffatome im Molekül enthalten. Geeignete Dicarbonsäuren sind beispielsweise Bernsteinsäure, Maleinsäure, Adipinsäure, Glutarsäure, Korksäure, Sebacinsäure oder Terephthalsäure. Bei der Herstellung der Polyamidoamine kann man auch Mischungen von Dicarbonsäuren einsetzen, ebenso Mischungen aus mehreren Polyalkylenpolyaminen. Geeignete Polyalkylenpolyamine sind beispielsweise Diethylentriamin, Triethylentetramin, Tetraethylenpentamin, Dipropylentriamin, Tripropylentetramin, Dihexamethylentriamin, Aminopropylethylendiamin und Bis-aminopropylethylendiamin. Die Dicarbonsäuren und Polyalkylenpolyamine werden zur Herstellung der Polyamidoamine auf höhere Temperaturen erhitzt, z.B. auf Temperaturen in dem Bereich von 120 bis 220, vorzugsweise 130 bis 180°C. Das bei der Kondensation entstehende Wasser wird aus dem System entfernt. Bei der Kondensation kann man gegebenenfalls auch Lactone oder Lactame von Carbonsäuren mit 4 bis 8 C-Atomen einsetzen. Pro Mol einer Dicarbonsäure verwendet man beispielsweise 0,8 bis 1,4 Mol eines Polyalkylenpolyamins.

Weitere Aminogruppen enthaltende Polymere sind mit Ethylenimin gepfropfte Polyamidoamine. Sie sind aus den oben beschriebenen Polyamidoaminen durch Umsetzung mit Ethylenimin in Gegenwart von Säuren oder Lewis-Säuren wie Schwefelsäure oder Bortrifluoridetheraten bei Temperaturen von beispielsweise 80 bis 100°C erhältlich. Verbindungen dieser Art werden beispielsweise in der DE-B-24 34 816 beschrieben.

Auch die gegebenenfalls vernetzten Polyamidoamine, die gegebenenfalls noch zusätzlich vor der Vernetzung mit Ethylenimin gepfropft sind, kommen als kationische Polymere in Betracht. Die vernetzten, mit Ethylenimin gepfropften Polyamidoamine sind wasserlöslich und haben z.B. ein mittleres Molgewicht von 3000 bis 2 Million Dalton. Übliche Vernetzer sind z.B. Epichlorhydrin oder Bischlorhydrinether von Alkylenglykolen und Polyalkylenglykolen.

Als Polymere, die primäre und/oder sekundäre Amino- und/oder Ammoniumgruppen aufweisen, kommen auch Polyallylamine in Betracht. Polymerisate dieser Art werden erhalten durch Homopolymerisation von Allylamin, vorzugsweise in mit Säuren neutralisierter Form oder durch Copolymerisieren von Allylamin mit anderen monoethylenisch ungesättigten Monomeren, die oben als Comonomere für N-Vinylcarbonsäureamide beschrieben sind.

Die obengenannten kationischen Polymerisate haben z.B. K-Werte von 8 bis 300, vorzugsweise 15 bis 180 (bestimmt nach H. Fikentscher in 5 %iger wäßriger Kochsalzlösung bei 25°C und einer Polymerkonzentration von 0,5 Gew.-%). Bei einem pH-Wert von 4,5 haben sie beispielsweise eine Ladungsdichte von mindestens 1, vorzugsweise mindestens 4 mVal/g Polyelektrolyt.

Bevorzugt in Betracht kommende kationische Polymere sind Polyethylenimin, Vinylamineinheiten enthaltende Polymere, Lysineinheiten enthaltende Polymere oder deren Mischungen. Beispiele für bevorzugt in Betracht kommende kationische Polymere sind:

Polylysine mit M_{w} von 250 bis'250 000, vorzugsweise 500 bis 100.000 sowie Lysin-Cokondensate mit Molmassen M_{w} von 250 bis 250 000, wobei man als cokondensierbare Komponente z.B. Amine, Polyamine, Ketendimere, Lactame, Alkohole, alkoxylierte Amine, alkoxylierte Alkohole und/oder nichtproteinogene Aminosäuren einsetzt,

Vinylamin-Homopolymere, 1 bis 99 % hydrolysierte Polyvinylformamide, Copolymerisate aus Vinylformamid und Vinylacetat, Vinylalkohol, Vinylpyrrolidon oder Acrylamid jeweils mit Molmassen von 3.000 - 2.000 000,

Polyethylenimine, vernetzte Polyethylenimine oder amidierte Polyethylenimine, die jeweils Molmassen von 500 bis 3.000.000 haben,

Primäre und/oder sekundäre Aminogruppen enthaltende Polymere, die zur Verminderung der Formaldehyd-Emission und gegebenenfalls als Hydrophilierungsmittel mit den Melamin/Formaldehydharz-Schaumstoffen in Kontakt gebracht worden sind, können gegebenenfalls darauf vernetzt werden. Eine Vernetzung der mit primäre und/oder sekundäre Aminogruppen enthaltenden Polymeren behandelten Schaumstoffe erzielt man beispielsweise durch Reaktion mit mindestens bifunktionellen Vernetzern wie Epichlorhydrin, Bischlorhydrinethern von Polyalkylenglykolen, Polyepoxiden, multifunktionellen Estern, multifunktionellen Säuren wie Polycarbonsäuren, z.B. Oxalsäure, Bernsteinsäure, Adipinsäure, Weinsäure, Citronensäure, Polyacrylsäure, Polymethacrylsäure, Polyacrylamidomethylpropansulfonsäure, Copolymerisate aus Acrylsäure und Maleinsäure, Schwefelsäure, Phosphorsäure, Acrylsäure, Methacrylsäure, Maleinsäure, Maleinsäureanhydrid, Itaconsäure oder multifunktionellen (Meth)Acrylaten wie Methylenbisacrylamid, Ethylenglykoldiacrylat, Ethylenglykoldimethacrylat, Polyethylenglykoldiacrylate oder -dimethacrylate mit einem Molekulargewicht nach dem Zahlenmittel von 200 bis 4000, Diacrylate oder Dimethacrylate von Additionsprodukten von 2 bis 400 Mol Ethylenoxid an 1 Mol eines Diols mit mindestens 3 C-Atomen im Molekül. Weiterhin geeignet sind:

Acrylsäure, Methacrylsäure, Maleinsäure, Maleinsäureanhydrid oder Ester von mindestens bifunktionellen Carbonsäuren z. B. Oxalsäuredialkylester, Maleinsäuredialkylester, Fumarsäuredialkylester, Bernsteinsäuredialkylester und Malonsäuredialkylester.

Die Formaldehyd-Emission der nach dem erfindungsgemäßen Verfahren hergestellten hydrophilen, offenzelligen, elastischen Melamin/Formaldehydharz-Schaumstoffe beträgt nach der EU Norm EN ISO 14184-1 weniger als 100 mg/Formaldehyd /kg Schaumstoff. Diese Schaumstoffe emittieren meistens weniger als 20 mg Formaldehyd pro kg Schaumstoff unter den genannten Meßbedingungen.

Die erfindungsgemäßen Schaumstoffe auf Basis von Melamin/Formaldehydharzen werden in Hygieneartikeln zur Aufnahme, Verteilung und Immobilisierung von Körperflüssigkeiten, insbesondere von Blut, eingesetzt. Ihr hydrophiler Charakter erlaubt eine spontane Aufnahme wäßriger Körperflüssigkeiten. Durch die offenzellige Struktur wird ein schneller Transport ins Schaumstoffinnere gewährleistet. Bei den Hygieneartikeln, die die erfindungsgemäß zu verwendenden Schaumstoffe enthalten, handelt es sich im wesentlichen um Babywindeln, Inkontinenzprodukte, Damenhygieneartikel, Wundauflagen oder Wundverbände.

Die Schaumstoffe auf Basis Melamin/Formaldehydharz zum erfindungsgemäßen Einsatz auf dem Hygienesektor sind offenporig und hydrophil. Die Tropfenaufnahmegeschwindigkeit der erfindungsgemäßen Melamin/Formaldehyd-Schaumstoffe beträgt weniger als 5 Sekunden, bevorzugt weniger als 2 Sekunden, besonders bevorzugt weniger als 1 Sekunde.

Die offenzelligen, elastischen Schaumstoffe werden vorzugsweise als flächige Gebilde in Form von Schaumvliesen mit einer Dicke von 0,1 bis 10 mm, vorzugsweise 1 bis 5 mm in Hygieneprodukten wie Babywindeln, Inkontinenz- und Damenhygieneartikeln oder als Wundauflagen bzw. in Verbandmaterialien eingearbeitet. Die Dichte der Schaumstoffe beträgt beispielsweise 5 bis 200 g/l, vorzugsweise 10 bis 50 g/l. Die Schäume weisen vorzugsweise eine Stegstruktur auf, haben eine spezifische Oberfläche, ermittelt nach der BET-Methode, von mehr als 0,5 m²/g, z.B. 1 bis 7 m²/g, verfügen über eine Free Swell Capacity von mehr als 20 g/g, z.B. 80 bis 120 g/g und haben im nassen Zustand eine Zugfestigkeit von > 60 J/ m² , z.B. 100 bis 600 J/m².

Im allgemeinen liegt im Hygieneartikel eine Kombination aus flüssigkeitsundurchlässigem Backsheet, flüssigkeitsdurchlässigem Topsheet und absorbierender Zwischenschicht (absorbent core) vor. Derartige Hygieneartikel sind bekannt und beispielsweise in DE - G-92 18 991 und EP-A-0 689 818 beschrieben. Die absorbierende Zusammensetzung wird zwischen Topsheet und Backsheet fixiert. Optional können elastische Bündchen und selbstklebende Verschlüsse im Hygieneartikel integriert werden. Ein bevorzugter Aufbau eines Hygieneartikels ist beispielsweise aus der US-A-3,860,003 bekannt.

Beim Einsatz der hydrophilen, offenzelligen, elastischen Schaumstoffe in einem Hygieneartikel bestehen beispielsweise zwei Möglichkeiten der Gestaltung der Zwischenschicht:
1. Die Melamin/Formaldehyd-Schaumstoffschicht dient ohne weitere Schichten als absorbierende Zwischenschicht. Sie ist dann gleichzeitig Akquisitions- bzw. Akquisitions-/Distributions-Schicht und Speicherschicht.
2. Die absorbierende Zwischenschicht besteht aus (a) einer Melamin/Formaldehyd-Schaumstoffschicht, die als Akquisitions- bzw. Akquisitions-/Distributions-Schicht wirkt und (b) einer Speicherschicht, die 10 - 100 Gew.-% hochquellfähiges Hydrogel enthält.

Die Speicherschicht stellt entweder eine Schicht aus einem Hydrogel oder Kompositionen dar, die hochquellfähige Hydrogele enthalten oder an denen sie fixiert sind. Jede Komposition ist geeignet, die die hochquellfähigen Hydrogele aufnehmen und die darüberhinaus in die absorbierende Zwischenschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (airlaid web, wet laid web) oder aus synthetischen Polymerfasern (meltblown web, spunbonded web) oder aber aus einem Misch-Faserwerk aus Cellulosefasern und synthetischen Fasern besteht. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstige Gewebe, geschlossene oder offenporige Schäume, perforierte Filme, Elastomere oder Gewebe aus Fasermaterial zum Einsatz gelangen. Wenn die Speicherschicht aus einer Komposition von Schichten besteht, sollte das Schichtenmaterial eine Porenstruktur aufweisen, deren Porenabmessungen klein genug sind, um die hochquellfähigen Hydrogelpartikeln zurückzuhalten. Obige Beispiele zur Komposition der Speicherschicht schließen auch Laminate aus mindestens zwei Schichten mit ein, zwischen die hochquellfähige Hydrogele eingebaut und fixiert sein können.

Des weiteren kann die Speicherschicht aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In den obengenannten Kompositionen der Speicherschicht können die hochquellfähigen Hydrogele z.B. mit einem Gewichtsanteil von 10 bis 100 Gew.-%, bevorzugt von 40 bis 100 Gew.-% und insbesondere bevorzugt von 70 bis 100 Gew.-% vorliegen. Wenn die oben beschriebene Komposition der Speicherschicht eine Fasermatrix darstellt, so resultiert die absorbierende Zusammensetzung aus einer Mischung von Fasermaterialien und hochquellfähigen Hydrogelen.

Die Speicherschicht kann vielfältige Fasermaterialien enthalten, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie Polyethylenterephthalatfasern (DACRON® oder KO-DEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190ºC, bevorzugt zwischen 75ºC und 175ºC aufweisen. Bei diesen Temperaturen ist noch keine Schädigung der Cellulosefasern zu erwarten.

Die vorstehend beschriebenen Synthesefasern können beispielsweise eine Länge von 1 bis 200 mm und einem Durchmesser von 0,1 bis 100 Denier (Gramm pro 9 000 Meter) haben. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Fasern können verschiedene Formen haben , z.B. gewebeartige, schmale zylinderartige, geschnitten-/spaltgarnartige, stapelfaserartige oder endlosfaserartige.

Die Fasern in der erfindungsgemäßen absorbierenden Zusammensetzung können hydrophil oder hydrophob sein. Kombinationen aus beiden Faserformen sind möglich. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner aus 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90º ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zwischenschicht sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Be-' handlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikeln werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser : (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Polyacrylamid- Überzüge (beschrieben in U.S. Patent 3,556,932 oder als Handelsprodukt der Marke Parez® 631 NC, Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind C₂-C₈ Dialdehyde, C₂-C₈ Monoaldehyde mit saurer Funktionalität, und insbesondere C₂-C₉ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt, vgl. beispielsweise WO-A-91/11162. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Generell wird in der erfindungsgemäßen Verwendung als oder in der absorbierende(n) Zwischenschicht ein hydrophiliertes Vlies aus einem offenzelligem, elastischen Melamin/Formaldehydharzschaumstoff mit sehr niedrigen Formaldehydemissionen verwendet. Die Abmessungen (Dicke) der absorbierenden Zwischenschicht liegt bei Einsatz mit der Funktionalität als absorbent core im allgemeinen zwischen 0,5 und 10 mm, bevorzugt zwischen 1 bis 5 mm. Bei Einsatz als Aquisitions- und Distributionsschicht in Kombination mit einer Speicherschicht liegt die Dicke zwischen 0,1 und 10 mm; bevorzugt zwischen 0,5 und 3 mm.

Das Topsheet kann auf unterschiedlichem Wege hergestellt werden, wie beispielsweise als Woven, Non-woven, versponnenes oder gekämmtes Fasergemisch. Bevorzugt wird gekämmtes Fasergemisch eingesetzt, das thermisch zum Topsheet gebunden wird. Das Flächengewicht des Topsheets beträgt bevorzugt 18 bis 25 g/m², eine Zugfestigkeit von mindestens 400 g/cm im trockenen Zustand und 55 g/cm im nassen Zustand.

Als Backsheet werden gewöhnlich flüssigkeitsundurchlässige Materialien eingesetzt, wie beispielsweise Polyolefine (z. B. Polyethylen-Backsheets), um die Kleidung des Trägers vor einem eventuellen Leakage zu schützen.

Die einzelnen Schichten, aus denen die Hygieneartikel aufgebaut sind, werden nach bekannten Methoden, wie Verschmelzen der Schichten durch Wärmebehandlung, Zugabe von Schmelzklebern, Latexbindern usw., miteinander verbunden. Die absorbierende Zwischenschicht wird zwischen Topsheet und Backsheet positioniert.

### Meßmethoden

### Tropfenaufnahmegeschwindigkeit

Auf eine ca. 5 mm dicke Schaumstoffschicht wird mit Hilfe einer Pipette ein einzelner Tropfen einer 0.9 %igen Kochsalzlösung aufgesetzt und die Zeit bestimmt bis der Tropfen in den Schaumstoff eingedrungen ist. War die Aufnahmezeit < 5 sec, so wurde der Schaumstoff als hydrophil bewertet.

### Dichte

Jede geeignete gravimetrische Methode kann zur Dichtebestimmung des Schaumstoffes herangezogen werden. Ermittelt wird die Masse an festem Schaumstoff pro Volumeneinheit Schaumstoffstruktur. Ein Verfahren zur Dichteermittlung des Schaumstoffes ist in der ASTM Methode Nr. D 3574-86, Test A, beschrieben. Diese Methode wurde ursprünglich zur Dichtebestimmung von Urethanschäumen entwickelt, kann aber auch zu diesem Zweck herangezogen werden. Danach wird bei einer vorkonditionierten Probe, wie in der Methode beschrieben, bei 22 +/- 2 °C deren Trockenmasse und Volumen ermittelt. Volumenbestimmungen größerer Probenabmessungen werden unter Normaldruck durchgeführt.

### Free swell capacity (FSC)

Bei dieser Methode wird die freie Quellbarkeit des offenzelligen elastischen Melamin/Formaldehyd-Schaumstoffs bestimmt. Zur Bestimmung der FSC wird aus dem Schaumstoff-Muster eine Probe geeigneter Größe, z.B. eine Probe mit einer Fläche von ca. 1 cm x 1 cm, ausgeschnitten und gewogen. Die Probe wird für 30 Minuten in einen Überschuß von 0,9 gew.-%iger wäßriger NaCl-Lösung gegeben (mindestens 0,83 l Kochsalzlösung pro 1 g Schaumstoff), danach entnommen und nach einer Abtropfzeit von 10 Minuten (die Probe wird an einer Ecke aufgehängt, um ein Zusammenpressen zu vermeiden) gewogen, um die aufgenommene Flüssigkeitsmenge zu bestimmen.

### Akquisitionszeit

Der offenzellige elastische Melamin/Formaldehyd-Schaumstoff wird in 1,5 mm bzw. 2 mm bzw. 4 mm dicke Schichten geschnitten. Eine kommerziell verfügbare Windel wird vorsichtig aufgeschnitten, das als Akquisitionsmedium dienende high-loft entnommen und statt dessen die offenzellige elastische Melamin/Formaldehyd-Schaumstoffschicht eingelegt. Die Windel wird wieder verschlossen. Die Aufgabe von synthetischer Harnersatzlösung erfolgt durch eine Kunststoffplatte mit einem Ring in der Mitte (Innendurchmesser des Ringes 6,0 cm, Höhe 4,0 cm). Die Platte wird belastet mit zusätzlichen Gewichten, so daß die Gesamtbelastung der Windel 13,6 g/cm2 beträgt. Die Kunststoffplatte wird auf der Windel so plaziert, daß der Mittelpunkt der Windel gleichzeitig die Mitte des Aufgaberinges darstellt. Es werden dreimal 60 ml 0,9 Gew.-%ige Kochsalz-Lösung aufgegeben. Die Kochsalz-Lösung wird in einem Meßzylinder abgemessen und durch den Ring in der Platte in einem Schuß auf die Windel aufgegeben. Gleichzeitig mit der Aufgabe wird die Zeit gemessen, die zum kompletten Eindringen der Lösung in die Windel notwendig ist. Die gemessene Zeit wird als Akquisitionszeit 1 notiert. Danach wird die Windel mit einer Platte für 20 Minuten belastet, wobei die Belastung weiterhin bei 13,6 g/cm2 gehalten wird. Danach erfolgt die zweite Aufgabe der Flüssigkeit. Die gemessene Zeit wird als Akquisitionszeit 2 notiert. Bei der Bestimmung von Akquisitionszeit 3 wird auf die gleiche Weise verfahren.

### Spezifische Oberfläche

Die Ermittlung der spezifischen Oberfläche erfolgt nach der BET-Methode gemäß DIN 66 132.

### Formaldehyd-Emission

Die Bestimmung erfolgte nach der Edana-Methode 210.1-99 (Prüfung nach EU Norm EN ISO 14184-1)

1g der zu untersuchenden Schaumprobe wird in kleine Stücke geschnitten zusammen mit 100 ml Wasser in einen Erlenmeyerkolben gefüllt und dicht verschlossen. Der Erlenmeyerkolben wird in ein auf 40°C temperiertes Wasserbad gesetzt und dort unter regelmäßigem Schütteln für 60 min belassen. Anschließend wird die erhaltene Lösung abfiltriert bzw. der Schaum ausgedrückt. In der erhaltenen Lösung wird der Formaldehydgehalt nach dem Acetylaceton-Verfahren bestimmt.

Sofern aus dem Zusammenhang nichts anderes hervorgeht, bedeuten die Prozentangaben in den Beispielen Gewichtsprozent. Die Molmassen M der Polymerisate wurden durch Lichtstreuung ermittelt. Die K-Werte
der Polymerisate wurden bestimmt nach H. Fikentscher, CelluloseChemie, Band 13, 58-64 und 71-74 (1932) bei einer Temperatur von 25°C und den in Tabelle 1 für die einzelnen Polymerisate jeweils angegebenen Konzentrationen, pH-Werten und Lösemitteln:

**Tabelle 1**

| Polymer | Lösemittel | Konzentration der Lösung [Gew%] | pH der Lösung |
|---|---|---|---|
| Polyvinylamin | 3%ige wässrige NaCl-Lösung | 0,5 | 11,0 |
| Polyacrylsäure | Wasser | 1,0 | 7,0 (neutralisert mit NaOH) |
| Polylysin | Wasser | 1,0 | 10,3 |

### Beispiele

### Vergleichsbeispiel 1

75 Teile eines sprühgetrockneten Melamin/Formaldehyd-Vorkondensates (Molverhältnis 1:3) wurden in 25 Teilen Wasser gelöst. Dieser Harzlösung wurden 3 % Ameisensäure, 2 % eines Na- C₁₂/C₁₈-Alkansulfonats und 19 % Pentan, jeweils bezogen auf das Harz, zugesetzt. Die Mischung wurde kräftig gerührt und anschließend in einer Schäumform aus Polypropylen durch Einstrahlung von Mikrowellenenergie bei 2,54 GHz verschäumt. Der Schaumstoff wurde bei 100°C getrocknet und anschließend bei 220°C 30 min getempert. Der so hergestellte Melamin/Formaldehyd -Schaumstoff war hydrophil und hatte eine Dichte von 10 g/l. Die nach 1 h Lagerung bei 40°C in Wasser gemessene Formaldehyd-Emission betrug 150 mg Formaldehyd / kg Schaumstoff. Die freie Aufnahmekapazität (FSC), betrug 103 g/g. Der Schaustoff hatte eine spezifische Oberfläche, bestimmt nach BET-Methode, von 5,3 m²/g.

### Vergleichsbeispiel 2

70 Teile eines sprühgetrockneten Melamin/Formaldehyd-Vorkondensates (Molverhältnis 1:1,6) wurden in 30 Teilen Wasser gelöst. Dieser Harzlösung wurden 3 % einer Emulgatormischung aus einem Alkanolamid und einem ethoxylierten Fettalkohol sowie 3 % Ameisensäure umd 10 % Pentan zugesetzt. Die Mischung wurde verschäumt, der Schaum getrocknet und getempert wie im Vergleichsbeispiel 1 beschrieben. Der auf diese Weise hergestellte Schaumstoff war hydrophob, seine Dichte betrug ebenfalls 10 g/l. Die Formaldehyd-Emission betrug weniger als 20 mg Formaldehyd / kg Schaumstoff.

### Beispiel 1

Aus dem nach Vergleichsbeispiel 1 hergestellten Schaumstoff wurden ca. 3 mm dicke Schichten geschnitten und durch Aufsprühen einer wäßrigen Lösung von Polyvinylamin mit 1,6% Polyvinylamin (K-Wert 90) beschichtet und anschließend bei 85°C im Vakuum getrocknet. Danach wurde die Formaldehyd-Emission des so erhaltenen Schaumstoffs gemessen. Die Meßergebnisse sind in Tabelle 2 angegeben.

### Beispiel 2

Aus dem nach Vergleichsbeispiel 1 hergestellten Schaumstoff wurden ca. 3 mm dicke Schichten geschnitten und durch Aufsprühen einer wäßrigen Lösung von Polyvinylamin mit 3,2% Polyvinylamin (K-Wert 90) beschichtet und anschließend bei 85°C im Vakuum getrocknet. Danach wurde die Formaldehyd-Emission des so erhaltenen Schaumstoffs gemessen. Die Meßergebnisse sind in Tabelle 2 angegeben.

### Beispiel 3

Aus dem nach Vergleichsbeispiel 1 hergestellten Schaumstoff wurden ca. 3 mm dicke Schichten geschnitten und durch Tauchen in einer 1%ige wäßrige Polyvinylamin-Lösung mit Polyvinylamin (K-Wert 105) beschichtet und anschließend bei 150°C im Vakuum getrocknet. Danach wurde die Formaldehyd-Emission des so erhaltenen Schaumstoffs gemessen. Die Meßergebnisse sind in Tabelle 2 angegeben.

### Beispiel 4

Aus dem nach Vergleichsbeispiel 1 hergestellten Schaumstoff wurden ca. 3 mm dicke Schichten geschnitten und durch Aufsprühen einer wäßrigen Lösung eines Gemisches von Polyvinylamin und Ethylenglycolbisglycidylether im Verhältnis 40:1 mit 3,2%, bezogen auf die Festsubstanzen des Gemisches, beschichtet und anschließend bei 85°C im Vakuum getrocknet. Danach wurde die Formaldehyd-Emission des so erhaltenen Schaumstoffs gemessen. Die Meßergebnisse sind in Tabelle 2 angegeben.

### Beispiel 5

Aus dem nach Vergleichsbeispiel 1 hergestellten Schaumstoff wurden ca. 3 mm dicke Schichten geschnitten und durch Aufsprühen einer wäßrigen Lösung eines zu 50 mol% mit HCl neutralisierten Polyvinylamins (K-Wert 90) beschichtet und anschließend bei 85°C im Vakuum getrocknet. Danach wurde die Formaldehyd-Emission des so erhaltenen Schaumstoffs gemessen. Die Meßergebnisse sind in Tabelle 2 angegeben.

### Beispiel 6

Aus dem nach Vergleichsbeispiel 1 hergestellten Schaumstoff wurden ca. 3 mm dicke Schichten geschnitten und durch Tauchen in eine 1%ige wäßrige Lösung von Polylysin (K-Wert 17) mit Polylysin beschichtet und anschließend bei 150°C im Vakuum getrocknet. Danach wurde die Formaldehyd-Emission des so erhaltenen Schaumstoffs gemessen. Die Meßergebnisse sind in Tabelle 2 angegeben.

**Tabelle 2**

| Beispiel-Nr. | Beschichtungsmittel | Freigesetzte Menge Form-aldehyd [ppm] |
|---|---|---|
| Vergleich 1 | | 150 |
| 1 | 1,6 % Polyvinylamin, K-Wert 90 | 25 |
| 2 | 3,2 % Polyvinylamin, K-Wert 90 | 14 |
| 3 | Polyvinylamin, K-Wert 105 | <10 |
| 4 | 3,2 % Reaktionsgemisch aus Polyvinylamin (K-Wert 90) und Ethylenglycoldiglycidylether im Verhältnis 40/1 | 28 |
| 5 | 3,2% Polyvinylamin (K-Wert 90) mit HCl zu 50 mol% neutralisiert | 21 |
| 6 | Polylysin, K-Wert 17 | 15 |

### Beispiel 7

Aus dem nach Vergleichsbeispiel 2 hergestellten Schaumstoff wurden ca. 3 mm dicke Schichten geschnitten und durch Aufsprühen einer wäßrigen Polyvinylamin-Lösung mit 6,4 % Polyvinylamin (K-Wert 90) beschichtet und anschließend bei 150°C im Vakuum getrocknet. Danach wurde die Formaldehyd-Emission des so erhaltenen Schaumstoffs gemessen. Die Meßergebnisse sind in Tabelle 3 angegeben.

### Beispiel 8

Aus dem nach Vergleichsbeispiel 2 hergestellten Schaumstoff wurden ca. 3 mm dicke Schichten geschnitten und durch Aufsprühen einer wäßrigen Polyvinylamin-Lösung mit 6,4 % Polyvinylamin (K-Wert 105) beschichtet und anschließend bei 150°C im Vakuum getrocknet. Danach wurde die Formaldehyd-Emission des so erhaltenen Schaumstoffs gemessen. Die Meßergebnisse sind in Tabelle 3 angegeben.

### Beispiel 9

Aus dem nach Vergleichsbeispiel 2 hergestellten Schaumstoff wurden ca. 3 mm dicke Schichten geschnitten und durch Tauchen in einer 1%igen wäßrigen Polylysin-Lösung mit Polylysin (K-Wert 17) beschichtet und anschließend bei 150°C im Vakuum getrocknet. Danach wurde die Formaldehyd-Emission des so erhaltenen Schaumstoffs gemessen. Die Meßergebnisse sind in Tabelle 3 angegeben.

### Beispiel 10

Aus dem nach Vergleichsbeispiel 2 hergestellten Schaumstoff wurden ca. 3 mm dicke Schichten geschnitten und durch Tauchen in einer 1%igen wäßrigen Polyethylenimin-Lösung mit Polyethylenimin (Molmasse M 25000) beschichtet und anschließend bei 85°C im Vakuum getrocknet. Danach wurde die Formaldehyd-Emission des so erhaltenen Schaumstoffs gemessen. Die Meßergebnisse sind in Tabelle 3 angegeben.

### Beispiel 11

Aus dem nach Vergleichsbeispiel 2 hergestellten Schaumstoff wurden ca. 3 mm dicke Schichten geschnitten und durch Aufsprühen eines wäßrigen Gemisches aus Polyvinylamin und Ethylenglycolbisglycidylether im Verhältnis 40:1 mit 3,2%, bezogen auf die Feststoffe des Gemisches, beschichtet und anschließend bei 85°C im Vakuum getrocknet. Danach wurde die Formaldehyd-Emission des so erhaltenen Schaumstoffs gemessen. Die Meßergebnisse sind in Tabelle 3 angegeben.

### Beispiel 12

Aus dem nach Vergleichsbeispiel 2 hergestellten Schaumstoff wurden ca. 3 mm dicke Schichten geschnitten und wurden durch Tauchen in einer 1%igen wäßrigen Polyvinylamin-Lösung mit Polyvinylamin (K-Wert 90) beschichtet und bei 150°C im Vakuum getrocknet. Anschließend wurden die so behandelte Schaumsstoff-Probe durch Aufsprühen einer wässrigen Lösung von Polyacrylsäure mit 4% Polyacrylsäure (K-Wert 110) beschichtet und bei 85°C im Vakuum getrocknet. Danach wurde die Formaldehyd-Emission des so erhaltenen Schaumstoffs gemessen. Die Meßergebnisse sind in Tabelle 3 angegeben.

**Tabelle 3**

| Beispiel | Beschichtungsmittel | Freigesetzte Menge Form-aldehyd [ppm] | Hydrophil | Hydrophob |
|---|---|---|---|---|
| Vergl. 2 | - | 15 | | X |
| 7 | Polyvinylamin, K-Wert 90 | <5 | X | |
| 8 | 6.4% Polyvinylamin, K-Wert 105 | <5 | X | |
| 9 | Polylysin, K-Wert 17 | <5 | X | |
| 10 | Polyethylenimin, MG 25000 | <5 | X | |
| 11 | 3.2 % Reaktionsgemisch aus Polyvinyl-amin (K-Wert 90) und Ethylenglycol-diglycidylether im Verhältnis 40/1 | <5 | X | |
| 12 | Polyvinylamin, K-Wert 90 und anschließend Polyacrylsäure K-Wert 110 | <5 | X | |

Die in Tabelle 3 zusammengestellten Daten lassen erkennen, daß durch die Beschichtung der Schaumstoffe mit primären und/oder sekundären Polyaminen die notwendige Hydrophilie gewährleistet und gleichzeitig die Formaldehyd-Emission weiter reduziert werden kann.

### Beispiel 13

Der nach Beispiel 8 behandelte Melamin-Formaldehydharz-Schaumstoff wurde in 2mm dicke Schichten geschnitten. Eine kommerziell verfügbare Windel wurde vorsichtig aufgeschnitten, das high-loft entnommen und statt dessen die 2mm dicke Schaumstoffschicht in die Windel eingelegt. Danach wurde die Windel wieder verschlossen und die Zeiten bestimmt, die für die Aufnahme von 3 aufeinanderfolgenden Zugaben von jeweils 60 ml synthetischem Urin (0,9%ige wäßrige Kochsalzlösung) benötigt wurden. Die Meßwerte sind in Tabelle 4 angegeben.

### Beispiel 14

Beispiel 13 wurde mit der Ausnahme wiederholt, daß man einen nach Beispiel 9 hergestellten Schaum in 2 mm dicke Schichten schnitt und die 2mm dicken Schichten in eine Windel einarbeitete. Die Meßwerte sind in Tabelle 4 angegeben.

### Vergleichsbeispiel 3

Eine kommerziell verfügbare Windel wurde vorsichtig aufgeschnitten, das high-loft entnommen, anschließend wieder eingelegt und die Windel wiederum verschlossen. Diese Vorgehensweise sollte eine optimale Vergleichbarkeit sicherstellen. Anschließend wurden die Akquisitionszeiten bestimmt. Sie sind in Tabelle 4 angegeben.

### Vergleichsbeispiel 4

Der nach Vergleichsbeispiel 2 hergestellte formaldehydarme Schaum aus Melamin/Formaldehyd-Kondensat wurde, wie im Beispiel 13 beschrieben, in eine Windel eingearbeitet. Anschließend bestimmte man die Akquisitionszeiten für die Aufnahme von synthetischem Urin. Sie sind in Tabelle 4 angegeben.

### Vergleichsbeispiel 5

Der nach Vergleichsbeispiel 1 hergestellte Schaum aus Melamin/Formaldehyd-Kondensat wurde, wie im Beispiel 13 beschrieben, in eine Windel eingearbeitet. Anschließend bestimmte man die Akquisitionszeiten für die Aufnahme von synthetischem Urin. Sie sind in Tabelle 4 angegeben.

**Tabelle 4**

| Windel | Zeit für die Aufnahme der ersten 60 ml [sec] | Zeit für die Aufnahme der zweiten 60 ml [sec] | Zeit für die Aufnahme der dritten 60 ml [sec] |
|---|---|---|---|
| Vergleichsbeispiel 3 | 7 | 19 | 29 |
| Vergleichsbeispiel 4 | 80 | 125 | 148 |
| Vergleichsbeispiel 5 | 3 | 6 | 8 |
| Beispiel 13 | 4 | 6 | 9 |
| Beispiel 14 | 3 | 5 | 8 |

Die Tabelle 4 läßt erkennen, daß die Akquisitionszeiten des formaldehydarmen, mit primären und/oder sekundären Aminogruppen enthaltenden Polymeren beschichteten Schaums signifikant besser sind als die einer kommerziell erhältlichen Windel und auf dem gleichen Niveau liegen wie bei dem Schaumstoff gemäß Vergleichsbeispiel 1.

## Patentansprüche

1. Hydrophile, offenzellige, elastische Schaumstoffe aus Melamin/Formaldehyd-Harzen, **dadurch gekennzeichnet, daß** sie erhältlich sind durch
(a) Erhitzen und Vernetzen einer wäßrigen Lösung oder Dispersion, die jeweils mindestens ein Melamin/Formaldehyd-Vorkondensat, einen Emulgator, ein Treibmittel und einen Härter enthalten, unter Ausbildung eines Schaumstoffs,
(b) anschließendes Tempern des Schaumstoffs während einer Zeit von 1 bis 180 Minuten bei 120 bis 300°C, wobei flüchtige Anteile entfernt werden, und
(c) Behandeln des Schaumstoffs während des Temperns oder danach mit mindestens einem Polymeren, das primäre und/oder sekundäre Aminogruppen enthält und eine Molmasse von mindestens 300 hat.

2. Hydrophile, offenzellige, elastische Schaumstoffe nach Anspruch 1, **dadurch gekennzeichnet, daß** man ein Melamin/Formaldehyd-Vorkondensat einsetzt, bei dem das Molverhältnis Melamin zu Formaldehyd 1 : 1,0 bis 1 : 5,0 beträgt.

3. Hydrophile, offenzellige, elastische Schaumstoffe nach Anspruch 1 oder 2, **dadurch gekennzeichnet, daß** man ein Melamin/Formaldehyd-Vorkondensat einsetzt, bei dem das Molverhältnis Melamin zu Formaldehyd 1 : 2,0 bis 1 : 5,0 beträgt.

4. Hydrophile, offenzellige, elastische Schaumstoffe nach einem der Ansprüche 1 bis 3, **dadurch gekennzeichnet, daß** man ein Melamin/Formaldehyd-Vorkondensat einsetzt, bei dem das Molverhältnis Melamin zu Formaldehyd 1: 2,5 bis 1: 3,5 beträgt.

5. Hydrophile, offenzellige, elastische Schaumstoffe nach einem der Ansprüche 1 bis 4, **dadurch gekennzeichnet, daß** man als Polymere primäre und/oder sekundäre Amino- und/oder Ammoniumgruppen enthaltende Polymere mit einer Molmasse von 500 bis 5 Millionen einsetzt.

6. Hydrophile, offenzellige, elastische Schaumstoffe nach einem der Ansprüche 1 bis 5, **dadurch gekennzeichnet, daß** die Molmasse der Polymeren 1000 bis 100 000 beträgt.

7. Hydrophile, offenzellige, elastische Schaumstoffe nach einem der Ansprüche 1 bis 6, **dadurch gekennzeichnet, daß** man als Polymere Vinylamineinheiten enthaltende Polymerisate, Polyethylenimine, Lysinkondensate und/oder Polyallylamine einsetzt.

8. Verfahren zur Herstellung von hydrophilen, offenzelligen, elastischen Schaumstoffen aus Melamin/Formaldehyd-Harzen, **dadurch gekennzeichnet, daß** man
(a) eine wäßrige Lösung oder Dispersion, die jeweils mindestens ein Melamin/Formaldehyd-Vorkondensat, einen Emulgator, ein Treibmittel und einen Härter enthalten, unter Ausbildung eines Schaumstoffs und Vernetzen des Vorkondensates erhitzt,
(b) den Schaumstoff dann während einer Zeit von 1 bis 180 Minuten bei 120 bis 300°C tempert, wobei flüchtige Anteile entfernt werden, und
(c) ihn während des Temperns oder danach mit mindestens einem Polymeren behandelt, das primäre und/oder sekundäre Aminogruppen enthält und eine Molmasse von mindestens 300 hat.

9. Verfahren nach Anspruch 8, **dadurch gekennzeichnet, daß** man
(a) ein Melamin/Formaldehyd-Vorkondensat einsetzt, bei dem das Molverhältnis Melamin zu Formaldehyd 1 : 2,0 bis 1 : 5,0 beträgt.

10. Verfahren nach Anspruch 8 oder 9, **dadurch gekennzeichnet, daß** man (a) ein Melamin/Formaldehyd-Vorkondensat einsetzt, bei dem das Molverhältnis Melamin zu Formaldehyd 1 : 2,5 bis 1 : 3,5 beträgt.

11. Verfahren nach einem der Ansprüche 8 bis 10, **dadurch gekennzeichnet, daß** man als Polymere Vinylamineinheiten enthaltende Polymerisate, Polyethylenimine, Lysinkondensate und/oder Polyallylamine einsetzt.

12. Verfahren nach einem der Ansprüche 8 bis 11, **dadurch gekennzeichnet, daß** daß die Polymerisate eine Molmasse von 500 bis 5 Millionen haben.

13. Verfahren nach einem der Ansprüche 8 bis 12, **dadurch gekennzeichnet, daß** die Polymerisate eine Molmasse von 1000 bis 100 000 haben.

14. Verfahren nach einem der Ansprüche 8 bis 13, **dadurch gekennzeichnet, daß** man als Polymere Vinylamineinheiten enthaltende Polymere einsetzt

15. Verfahren nach einem der Ansprüche 8 bis 14, **dadurch gekennzeichnet, daß** man als Polymere Polyethylenimine einsetzt.

16. Verwendung der hydrophilen, offenzelligen, elastischen Schaumstoffe aus Melamin/Formaldehyd-Harzen nach den Ansprüchen 1 bis 7 in Hygieneartikeln zur Aufname, Verteilung und Immobilisierung von Körperflüssigkeiten.

17. Verwendung nach Anspruch 16, **dadurch gekennzeichnet, daß** es sich bei den Hygieneartikeln um Babywindeln, Inkontinenzprodukte, Damenhygieneartikel, Wundauflagen oder Wundverbände handelt.

## Claims

1. Hydrophilic open-celled resilient foams comprising melamine-formaldehyde resins, obtainable by
(a) heating and crosslinking an aqueous solution or dispersion each containing at least a melamine-formaldehyde precondensate, an emulsifier, a blowing agent and a curing agent to form a foam,
(b) then conditioning the foam at from 120 to 300°C for from 1 to 180 minutes to remove volatiles, and
(c) treating the foam during the conditioning or thereafter with at least one polymer comprising primary and/or secondary amino groups and having a molar mass of not less than 300.

2. The hydrophilic open-celled resilient foams according to claim 1, **characterized in that** the melamine-formaldehyde precondensate used has a molar ratio of melamine to formaldehyde in the range from 1:1.0 to 1:5.0.

3. The hydrophilic open-celled resilient foams according to claim 1 or 2, **characterized in that** the melamine-formaldehyde precondensate used has a molar ratio of melamine to formaldehyde in the range from 1:2.0 to 1:5.0.

4. The hydrophilic open-celled resilient foams according to any of claims 1 to 3, **characterized in that** the melamine-formaldehyde precondensate used has a molar ratio of melamine to formaldehyde in the range from 1:2.5 to 1:3.5.

5. The hydrophilic open-celled resilient foams according to any of claims 1 to 4, **characterized in that** at least one polymer used comprises polymers comprising primary and/or secondary amino and/or ammonium groups and having a molar mass of from 500 to 5 million.

6. The hydrophilic open-celled resilient foams according to any of claims 1 to 5, **characterized in that** the molar mass of the polymers is in the range from 1000 to 100,000.

7. The hydrophilic open-celled resilient foams according to any of claims 1 to 6, **characterized in that** at least one polymer used comprises vinylamine polymers, polyethyleneimines, lysine condensates and/or polyallylamines.

8. A process for preparing hydrophilic open-celled resilient foams comprising melamine-formaldehyde resins, **characterized in that** it comprises
(a) heating and crosslinking an aqueous solution or dispersion each comprising at least a melamine-formaldehyde precondensate, an emulsifier, a blowing agent and a curing agent to form a foam,
(b) then conditioning the foam at from 120 to 300°C for from 1 to 180 minutes to remove volatiles, and
(c) treating the foam during the conditioning or thereafter with at least one polymer comprising primary and/or secondary amino groups and having a molar mass of not less than 300.

9. The process according to claim 8, **characterized in that**
(a) the melamine-formaldehyde precondensate used has a molar ratio of melamine to formaldehyde in the range from 1:2.0 to 1:5.0.

10. The process according to claim 8 or 9, **characterized in that** (a) the melamine-formaldehyde precondensate used has a molar ratio of melamine to formaldehyde in the range from 1:2.5 to 1:3.5.

11. The process according to any of claims 8 to 10, **characterized in that** at least one polymer used comprises vinylamine polymers, polyethyleneimines, lysine condensates and/or polyallylamines.

12. The process according to any of claims 8 to 11, **characterized in that** the polymers have a molar mass of from 500 to 5 million.

13. The process according to any of claims 8 to 12, **characterized in that** the polymers have a molar mass of from 1000 to 100,000.

14. The process according to any of claims 8 to 13, **characterized in that** the polymers used comprise vinylamine units.

15. The process according to any of claims 8 to 14, **characterized in that** the polymers used are polyethyleneimines.

16. The use of the hydrophilic open-celled resilient foams comprising melamine-formaldehyde resins according to any of claims 1 to 7 in hygiene articles to acquire, distribute and immobilize body fluids.

17. The use according to claim 16, **characterized in that** the hygiene articles are infant diapers, incontinence products, femcare articles, wound contact materials or secondary wound dressings.

## Revendications

1. Produits alvéolaires hydrophiles, élastiques et à cellules ouvertes en résines de mélamine/formaldéhyde, **caractérisés en ce qu'**on les obtient par :
(a) chauffage et réticulation d'une solution ou dispersion aqueuse qui contient chaque fois au moins un produit précondensé de mélamine/formaldéhyde, un émulsionnant, un agent moussant et un agent de durcissement, avec formation d'un produit alvéolaire,
(b) mise à température subséquente du produit alvéolaire pendant une durée de 1 à 180 minutes entre 120 et 300°C, les parties volatiles étant à cette occasion éliminées, et
(c) manipulation du produit alvéolaire pendant la mise à température ou après avec au moins un polymère qui contient des groupes amino primaires et/ou secondaires et a une masse molaire d'au moins 300.

2. Produits alvéolaires hydrophiles, élastiques et à cellules ouvertes selon la revendication 1, **caractérisés en ce qu'**on met en oeuvre un produit précondensé de mélamine/formaldéhyde dans lequel le rapport molaire mélamine formaldéhyde est de 1:1,0 à 1:5,0.

3. Produits alvéolaires hydrophiles, élastiques et à cellules ouvertes selon la revendication 1 ou 2, **caractérisés en ce qu'**on met en oeuvre un produit précondensé de mélamine/formaldéhyde dans lequel le rapport molaire mélamine formaldéhyde est de 1:2,0 à 1:5,0.

4. Produits alvéolaires hydrophiles, élastiques et à cellules ouvertes selon l'une quelconque des revendications 1 à 3, **caractérisés en ce qu'**on met en oeuvre un produit précondensé de mélamine/formaldéhyde dans lequel le rapport molaire mélamine formaldéhyde est de 1:2,5 à 1:3,5-.

5. Produits alvéolaires hydrophiles, élastiques et à cellules ouvertes selon l'une quelconque des revendications 1 à 4, **caractérisés en ce qu'**on met en oeuvre comme polymères des polymères contenant des groupes primaires et/ou secondaires amino et/ou ammonium présentant une masse molaire de 500 à 5 millions.

6. Produits alvéolaires hydrophiles, élastiques et à cellules ouvertes selon l'une quelconque des revendications 1 à 5, **caractérisés en ce que** la masse molaire des polymères est de 1000 à 100 000.

7. Produits alvéolaires hydrophiles, élastiques et à cellules ouvertes selon l'une quelconque des revendications 1 à 6, **caractérisés en ce qu'**on met en oeuvre comme polymères des polymères contenant des unités de vinylamine, des polyéthylénimines, des produits condensés de lysine et/ou des polyallylamines.

8. Procédé de fabrication de produits alvéolaires hydrophiles, élastiques et à cellules ouvertes en résines de mélamine/formaldéhyde, **caractérisé en ce que** :
(a) on chauffe une solution ou une dispersion aqueuse qui contient chaque fois au moins un produit précondensé de mélamine/formaldéhyde, un émulsionnant, un agent moussant et un agent de durcissement, avec formation d'un produit alvéolaire, et réticulation du produit précondensé,
(b) on met ensuite le produit alvéolaire à température pendant une durée de 1 à 180 minutes entre 120 et 300°C, les parties volatiles étant à cette occasion éliminées, et
(c) on le manipule pendant la mise à température ou après avec au moins un polymère qui contient des groupes amino primaires et/ou secondaires et a une masse molaire d'au moins 300.

9. Procédé selon la revendication 8, **caractérisé en ce que** :
(a) on met en oeuvre un produit précondensé de mélamine/formaldéhyde dans lequel le rapport molaire mélamine formaldéhyde est de 1:2,0 à 1:5,0.

10. Procédé selon la revendication 8 ou 9, **caractérisé en ce qu'**on met en oeuvre (a) un produit précondensé de mélamine/formaldéhyde dans lequel le rapport molaire mélamine formaldéhyde est de 1:2,5 à 1:3, 5.

11. Procédé selon l'une quelconque des revendications 8 à 10, **caractérisé en ce qu'**on met en oeuvre comme polymères des polymères contenant des unités vinylamine, des polyéthylénimines, des produits condensés de lysine et/ou des polyallylamines.

12. Procédé selon l'une quelconque des revendications 8 à 11, **caractérisé en ce que** les polymères ont une masse molaire de 500 à 5 millions.

13. Procédé selon l'une quelconque des revendications 8 à 12, **caractérisé en ce que** les polymères ont une masse molaire de 1000 à 100 000.

14. Procédé selon l'une quelconque des revendications 8 à 13, **caractérisé en ce qu'**on met en oeuvre comme polymères des polymères contenant des unités vinylamine.

15. Procédé selon l'une quelconque des revendications 8 à 14, **caractérisé en ce qu'**on met en oeuvre comme polymères des polyéthylénimines.

16. Utilisation des produits alvéolaires hydrophiles, élastiques et à cellules ouvertes en résines de mélamine/formaldéhyde selon les revendications 1 à 7 dans des articles d'hygiène pour l'absorption, la répartition et l'immobilisation de fluides corporels.

17. Utilisation selon la revendication 16, **caractérisée en ce qu'**il s'agit, pour les articles d'hygiène, de langes pour bébés, de produits pour l'incontinence, d'articles d'hygiène féminine, de compresses pour plaies ou de bandages pour blessures.
